# EUROPEAN PATENT APPLICATION

(11) **EP 0 704 212 A2**
(43) Date of publication of application: **03.04.1996**
(21) Application number: 95810600.7
(22) Date of filing: 25.09.1995
(51) Int. Cl.: A61K 31/20, A61K 31/23, A23L 1/30, A23L 1/302, A23L 3/3481

(54) **Method of minimizing hepatic reperfusion injury**

(30) Priority: 26.09.1994 GB 9419345
(71) Applicant: SANDOZ NUTRITION LTD., CH-3001 Berne (CH)
(72) Inventor: Schneider, Heinz, CH-1792 Cordast (CH); Thurman, Ronald G., Capel Hill, NC 27516 (CH)
(74) Representative: Smolders, Walter

(57) **Abstract**

The present invention provides a method of minimizing hepatic reperfusion injury in a patient following surgery which comprises pre-operatively administering to a patient in need of such treatment omega-3 fatty acids in an amount sufficient to improve the hepatic micro-circulation, said omega-3 PUFAs being protected in a pharmacologically acceptable way against peroxidation.

## Description

This invention relates to a method of minimizing hepatic reperfusion injury employing omega-3 fatty acids, e.g. in the form of fish oil.

Previous studies have demonstrated protective effects of fish oil against certain tissue damage. Thus, it has been reported that fish oil diets attenuate myocardial dysfunction and injury caused by global ischemia and reperfusion in isolated rat hearts.

In contrast, it is known that fish oil enhances the deleterious effects of ethanol in the liver. The liver is different from other organs, in that it comprises many Kupffer cells.

The reperfusion injury in kidneys, heart and lungs occurs primarily through leucocyte activation. This is different in the liver, where it is believed that the reperfusion injury is primarily caused by the Kupffer cells and that leucocyte activation only exacerbates the necrotisation induced by the Kupffer cells.

It has now been found that it is possible to minimize hepatic reperfusion injury following surgery with the aid of omega-3 polyunsaturated fatty acids (PUFAs) if such acids are protected against peroxidation.

The invention therefore provides a method of minimizing hepatic reperfusion injury in a patient following surgery which comprises pre-operatively administering to a patient in need of such treatment omega-3 fatty acids in an amount sufficient to improve the hepatic micro-circulation, said omega-3 PUFAs being protected in a pharmacologically acceptable way against peroxidation.

The invention also provides the use of omega-3 PUFAs which are protected against peroxidation in the manufacture of a pre-operative diet for minimizing hepatic injury following surgery in humans.

Pharmacologically acceptable ways of protecting omega-3 PUFAs against peroxidation are known in the art. They include pharmacologically acceptable micro-encapsulation of omega-3 PUFAs and the use of pharmacologically acceptable antioxidants.

A typical example suitable for use as pharmacologically acceptable micro-encapsulation agents is starch. The micro-encapsulation can be effected in a manner known per se. The micro-encapsules may be coated in a manner known per se, by pharmacologically acceptable coating agents such as Gum Arabic.

Typical examples of antioxidants suitable for use in the method of the invention include antioxidant vitamins such as Vitamin C, Vitamin E or mixtures thereof.

The amount of antioxidant added should be sufficient to prevent peroxidation of the omega-3 PUFAs. Such amounts can be easily calculated. In general, for convenience, any antioxidants employed to prevent peroxidation, will be employed in excess. It will be appreciated that the presence of any other agent administered in association with the omega-3 PUFAs may require adjustment of the amount of antioxidant to be employed.

The omega-3 PUFAs may be employed in free acid form, in a form suitable for the physiological supply of omega-3 PUFAs, e.g. in triglyceride form, or in the form of pharmacologically acceptable natural sources of omega-3 PUFAs. Such natural sources include linseed oil and fish oils such as menhaden oil, salmon oil, mackeral oil, tuna oil, codliver oil and anchovy oil. Said natural sources, in particular, the fish oils, comprise substantial amounts of omega-3 fatty acids. Where the omega-3 PUFAs are employed in triglyceride form, said triglycerides may comprise esters with other pharmacologically acceptable fatty acids.

Preferred omega-3 PUFAs include eicosapentaenoic acid (EPA) and docosahexaenoic acid (DHA), in free acid form, in triglyceride form or in form of natural sources having a high EPA and/or DHA content.

The minimizing effect of omega-3 PUFAs protected against peroxidation ("protected" omega-3 PUFAs) on hepatic reperfusion injury following surgery is indicated by tests. A suitable test for this activity is i.a. the low-flow reflow rat liver perfusion model, which is disclosed and discussed hereinafter. The test results on rat livers show that omega-3 PUFAs which are protected against peroxidation, do not significantly influence malondialdehyde (MDA) production but do reduce lactic dehydrogenase (LDH) release during reperfusion when comparing the thus determined MDA and LDH levels with that determined on livers from control rats fed with a standard diet in which the omega-3 PUFAs protected against peroxidation is replaced by the same amount of corn oil. The (vs. control) improved hepatic microcirculation in rat livers treated with "protected" omega-3 PUFAs is i.a. illustrated by reduced portal pressure, by the shorter time for trypan blue to evenly distribute in the liver and by the improved bile production.

To attain the desired effect, the "protected" omega-3 PUFAs are conveniently administered in the form of complete diet or of a dietary supplement preferably the latter. The "protected" omega-3 PUFAs will conveniently be administered over a period of 3 days or longer, e.g. from 3 to 6 days prior to surgery.

The daily amount will i.a. be patient dependent. For adults, the total amount of "protected" omega-3 PUFAs to be supplied over the period of treatment will preferably not be below 15 g.

The daily amount of "protected" omega-3 PUFAs to be administered prior to surgery will conveniently lie in the range of from 2 g to 5 g. The daily amount may be administered in one or more serving, e.g. 2 to 4 servings per day.

It will be appreciated that "protected" omega-3 PUFAs may be administered in higher amounts than those indicated hereinabove, and that such higher amounts will in general not impair the desired effect or provoke undesired side effects.

The omega-3 PUFAs may also be employed in admixture with other fatty acids, including omega-6 and omega-9 PUFAs.

The omega-6 PFUAs may be in the free acid form or in a form suitable for the physiological supply of omega-6 PFUAs, e.g. in triglyceride form. Examples of omega-6 PFUAs particularly appropriate for use according to the invention include linoleic acid and arachidonic acid (ETA), linoleic acid being most preferred. Examples of suitable omega-6 PFUA sources are known in the art. They include vegetable oils. Preferred are omega-6 PFUA sources having a high linoleic acid content such as safflower oil, sunflower oil, soya oil, cotton oil and corn oil.

A preferred natural source for fatty acid mixtures comprising omega-9 PUFAs are fish oils.

The formulations for use in the method of the invention may comprise and preferably will comprise other nutritionally acceptable components such as vitamins, minerals, trace elements, fibers (preferably soluble fibers) as well as nitrogen sources, carbohydrate sources and additional fatty acid sources.

Said formulations may be formulated and administered in a form suitable for parenteral or enteral administration. The enteral administration route is preferred; particularly contemplated are oral administration, nasal administration and/or tube feeding. The formulations are conveniently administered in the form of an aqueous liquid. The formulation suitable for enteral application are accordingly preferably in aqueous form or in powder form, whereby the powder is conveniently added to water prior to use. For use as tube feeding, the amount of water to be added will i.a. depend on the patient's fluid requirements and condition.

Examples of suitable nitrogen sources include nutritionally acceptable proteins such as caseinates, or protein hydrolysates.

Examples of suitable carbohydrate sources include maltodextrins. Examples of suitable fatty acid energy supply sources include triglyceride sources.

Examples of vitamins suitable for incorporation in the composition of the invention include Vitamin A, Vitamin D, Vitamin E, Vitamin K, Vitamin C, folic acid, thiamin, riboflavin, Vitamin B₆, Vitamin B₁₂, niacin, biotin and panthotenic acid in pharmaceutically acceptable form.

Examples of mineral elements and trace elements suitable for incorporation in the composition of the invention include sodium, potassium, calcium, phosphorous, magnesium, manganese, copper, zinc, iron, selenium, chromium and molybdenum in pharmaceutically acceptable form.

In particular, the formulations for use in the method of the invention will preferably comprise beta-carotene (Vitamin A), Vitamin E, Vitamin C, thiamine, Vitamin B₁₂, choline, selenium and zinc in pharmaceutically acceptable form.

The term soluble fiber as used herein refers to fibers which are able to substantially undergo fermentation in the colon to produce short chain fatty acids. Examples of suitable soluble fibers include pectin, guar gum, locust bean gum, xanthan gum. They may be hydrolysed or not. For adults the total amount of soluble fibre per day will conveniently lie in the range of from 3 to 30 g/day.

Formulations for use in the method of the invention are primarily intended for use as a supplement. The amount of energy supplied by them should in such case not be too excessive, in order not to unnecessarily suppress the patients appetite. The supplement should conveniently comprise energy sources in an amount supplying from 600 to 1500 Kcal/day. The contribution of the nitrogen source, carbohydrate source and lipid source to the total daily caloric may vary within wide ranges. In preferred compositions of the invention the carbohydrate source provides for 40 to 70 % of the total energy supply and, the nitrogen and fatty acid source each for 15 to 30 % of the total energy supply of the composition.

Such formulations may be obtained in a manner known per se, e.g. by admixing the ingredients.

**The following Example illustrates the invention:**

### EXAMPLE - Effects of fish oil treatment on hepatic reperfusion injury in a low-flow reflow perfusion model

**Animals:** Male Sprague-Dawley rats weighing between 100-130 g were caged individually and given powdered diets containing 5 % of weight as corn oil or encapsulated fish oil in a blind design ad libitum for 13-15 days (Table 1 hereinafter). The diets were kept under nitrogen at 4° C, and fresh diets were provided daily. Food intake was assessed by weighing the remaining diet each day. Rats were fasted for 24 h prior to liver perfusion.

**Liver Perfusion:** Rats were anesthetized with pentobarbital sodium (50 mg) before surgery and livers were removed surgically and perfused via a cannula inserted into portal vein with Krebs-Henseleit bicarbonate buffer (pH 7.4, 37° C) saturated with an oxygen-carbon dioxide (95:5) mixture in a non-recirculating system. After surgery, livers were perfused at flow rates of about 1 ml/g/min for 75 minutes (low-flow). Subsequently, livers were perfused at normal flow rates (4 ml/g/min) for 40 minutes (reflow). Oxygen concentration in the effluent perfusate was monitored continuously with a Teflon-shielded, Clark-type oxygen electrode. Oxygen uptake was calculated from the influent minus effluent concentration difference, the flow rate and the liver wet weight.

**Portal Pressure and Bile Production:** A needle connected to a polyethylene tube (PE 90) was inserted into a cannula just prior to its entry into the portal vein. Portal pressure was monitored by changes in the height of a water column during perfusion.The common bile duct was cannulated with polyethylene tubing (PE-10; Clay Adams), and aliquots of bile were collected into tared vials at 15-min intervals in the low-flow and at 10-min intervals during reflow periods. Rates of bile production were calculated from weight of bile, time intervals and the liver wet weight.

**Lactate Dehydrogenase (LDH) and Malondialdehyde Determination (MDA):** LDH activity in the perfusate was determined using standard enzymatic techniques, and MDA was assessed using the thiobarbituric acid method (Jha H.V. et al; Inhibition of in vitro microsomal lipid peroxidation by isoflavonoids Biochem. Pharmacol. 1985; 34:1367-1369). Three ml of perfusate was mixed thoroughly with a reagent containing 15 % trichloroacetic acid, 0.375 % thiobarbituric acid and 0.25 N hydrochloric acid and was heated for 15 minutes in a boiling water bath. After cooling, samples were centrifuged at 1,000 x g for 10 minutes and the absorbance of the supernatant was determined at 535 nm. MDA concentration in perfusate was calculated by comparison with MDA standards. Rates of release of LDH and MDA were expressed per gram wet weight of the liver per hour.

**Trypan Blue Distribution Time:** To assess microcirculation in the liver, trypan blue was infused into the liver at the end of all experiments at final concentrations of 0.2 mM. The time for the liver surface to turn evenly dark blue was recorded.

**Statistical Analysis:** Student's T-test or ANOVA were used when appropriate. Difference will be considered significant at P<0.05 level.

### RESULTS

**Food Intake and Body Weight:** Average daily food intake was around 20 grams per day per rat in corn oil group and was not altered significantly by encapsulated fish oil. Body weights of rats increased gradually from about 120 to 245 grams, and average daily weight gain was around 9 grams per day per rat in both groups over the two week feeding period.

**Effects of Fish Oil-Treatment on Hepatocellular Damage in a Low-flow Reflow Model:** Rat livers were perfused at flow rates around 1 ml/g/min for 75 minutes followed by reperfusion for 40 minutes at flow rates around 4 ml/g/min. Release of lactate dehydrogenase rates of bile production, rates of malondialdehyde release and portal pressure during both the low-flow and reflow periods were determined.

During the low flow period, LDH release was minimal (around 1 IU/g/h at 75 minute) in livers from both corn oil and encapsulated fish-oil-treated rats. When the flow rate was increased to 4 ml/g/min, however, LDH release increased gradually reaching a new steady-state value in about 30 minutes. Maximal LDH release during the reperfusion period was around 50 IU/g/h in livers from control rats, but values were reduced significantly by encapsulated fish-oil treatment to around 16 IU/g/h. Rates of bile production were around 12 µ/g/h at the end of low-flow period in corn oil controls, and were not significantly different in the encapsulated fish-oil-treated group. Maximal bile production increased to about 23 µl/g/h during the reflow period in controls, but value reached 38 µl/g/h in livers from encapsulated fish-oil-treated rats. Oxygen uptake during reperfusion was 111 and 119 µmol/g/h in livers from control and encapsulated fish-oil-treated rats, respectively (p>0.05, student's t-test). Taken together, reperfusion injury, which occurs when oxygen was reintroduced into previously anoxic liver, was minimized by fish-oil treatment.

**Effects of Fish-Oil-Treatment on Malondialdehyde production:** MDA, an end product of lipid peroxidation, was released into the effluent perfusate at rates around 15 nmol/g/h during 75 minutes of low-flow perfusion in livers from both corn oil and encapsulated fish-oil-treated rats. When flow rates were restored to normal, MDA production increased rapidly to peak values in about 15 minutes and then decreased slightly. Maximal MDA production during the reperfusion period was around 90 nmol/g/h in control rats and 80 noml/g/h in encapsulated fish-oil-treated rats, respectively and values were not statistically significantly different.

**Effects of Fish-Oil-Treatment on Portal Pressure and Trypan Blue Distribution Time:** Portal pressure, an indicator of the hepatic microcirculation, was monitored during both low-flow and reflow periods. Portal pressure during the low-flow period was around 3 cm H₂O in livers from both corn oil and encapsulated fish-oil-treated rats. Values increased to 10.5 cm H2O during reperfusion in livers from corn-oil-treated-rats, but only reached 8.3 cm H₂O in livers from encapsulated fish-oil-treated rats. It took about 640 seconds for trypan blue to distribute evenly in livers from corn-oil-treated rats, values which were reduced by 44 % by encapsulated fish-oil-treatment.

**TABLE 1**

| **Composition of (corn oil based) Control and encapsulated Fish Oil Diets** | | |
|---|---|---|
| | **% (W/W)** | |
| | **control** | **fish oil** |
| Casein | 15.0 | 15.0 |
| Sucrose | 51.0 | 49.0 |
| Corn oil | 5.0 | ----- |
| Fish oil (encapsulated) | ----- | 5.0 |
| Cellulose | 5.0 | 5.0 |
| mineral mixture | 3.5 | 3.5 |
| Vitamin mixture | 1.0 | 1.0 |
| DL-methionine | 0.3 | 0.3 |
| Choline bitartrate | 0.2 | 0.2 |
| Corn starch | 15.0 | 16.0 |
| Gum Arabic | 4.0 | 5.0 |

The encapsulated fish-oil is in triglyceride form, with an EPA:DHA ratio of 18:12.

The fatty acid content is as follows:
- omega-3 fatty acids 35 %
   including
   - EPA 18 % of fish oil
   - DHA 12 % of fish oil
   - Docosapentaenoic acid (DPA) 2 % of fish oil
   - others 3 % of fish oil
- polyunsaturated fatty acids other than omega-3 11 %
- monounsaturated fatty acids 28 %
- saturated fatty acids 26 %
   (% are by weight)

The mineral mixture comprises:

### Mineral:

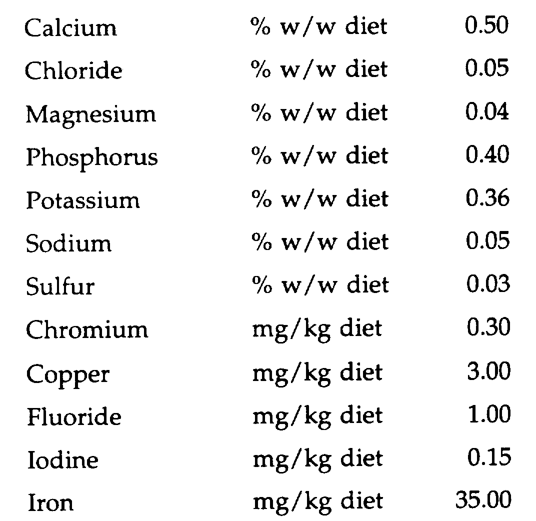

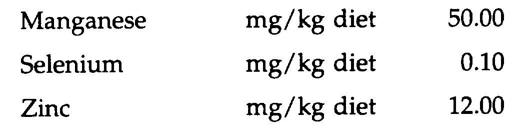

The vitamin mixture comprises pro kg diet:

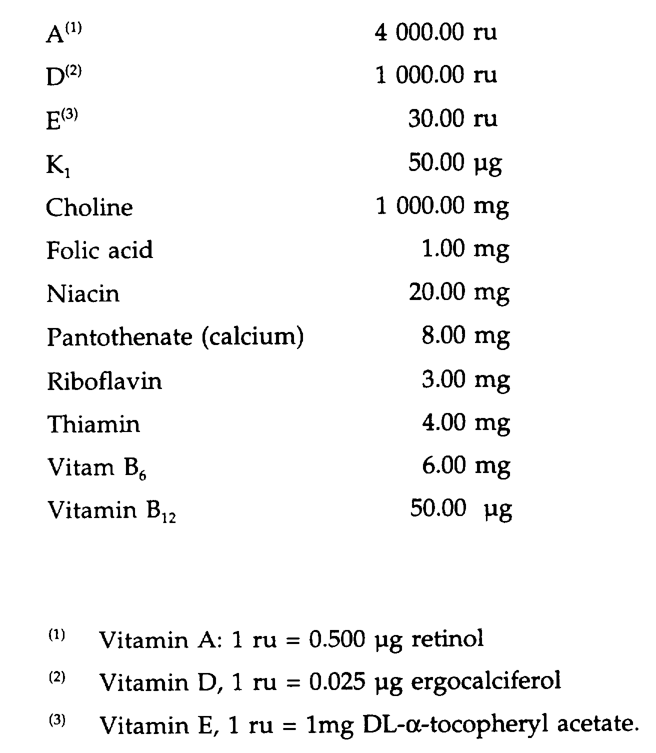

## Claims

1. The use of omega-3 fatty acids which are protected against peroxidation in the manufacture of a pre-operative diet for minimizing hepatic injury following surgery in humans.

2. The use of Claim 1, wherein the omega-3 PUFAs are encapsulated for protection against peroxidation.

3. The use of Claim 1, wherein the omega-3 PUFAs are protected against peroxidation with the aid of Vitamin C, Vitamin E or a mixture thereof.

4. The use of Claims 1 to 3, wherein the omega-3 PUFAs are in the form of fish-oil.
